# EUROPEAN PATENT SPECIFICATION

(11) **EP 2 355 883 B1**
(45) Date of publication and mention of the grant of the patent: **22.07.2015**
(21) Application number: 09756353.0
(22) Date of filing: 19.11.2009
(51) Int. Cl.: A61M 16/04

(54) **IMPROVED AIRWAY DEVICE**
VERBESSERTE ATEMWEGSVORRICHTUNG
DISPOSITIF AMÉLIORÉ DE VOIE AÉRIENNE

(30) Priority: 21.11.2008 GB 0821291; 23.09.2009 GB 0916706
(43) Date of publication of application: 17.08.2011
(73) Proprietor: Nasir, Muhammed Aslam, Bedfordshire LU1 4AX (GB)
(72) Inventor: Nasir, Muhammed Aslam, Bedfordshire LU1 4AX (GB)
(74) Representative: Instone, Alicia Claire
(86) International application number: PCT/GB2009/051574
(87) International publication number: WO 2010/058219

(56) References cited:
- EP-A2- 0 448 878
- EP-A2- 1 579 885
- EP-A2- 1 875 937
- WO-A1-01/13980
- WO-A1-03/018094
- WO-A2-03/020340
- US-A1- 2008 142 017

## Description

### Field of the Invention

The present invention relates to medical devices and is particularly applicable, but in no way limited, to laryngeal airway devices and to their methods of manufacture. This invention relates to an anatomically oriented, simple but versatile, improved airway device. It is particularly applicable, but in no way limited, to devices used in the administration of Oxygen and/or anaesthetic gases to a human or veterinary patient breathing spontaneously or for Intermittent Positive Pressure Ventilation (IPPV) during a surgical procedure or resuscitation.

### Background to the Invention

A wide variety of devices are known and are currently used in spontaneously breathing or for IPPV in anaesthetized patients, during recovery after anaesthetics, in weaning off a certain group of patients in intensive care, or during resuscitation to provide a clear and hands-free airway. A number of these devices are listed in the applicant's earlier patent GB2,393,399B (Nasir), the text of which is hereby imported by reference and which is intended to form an integral part of this disclosure. GB2,393,399B describes a new type of airway device which has a soft laryngeal cuff adapted to fit anatomically over, and form a seal with, the laryngeal structure of a patient.

The laryngeal cuffs on these devices are generally non-inflatable, but rather are formed from a soft, deformable material that can adapt to the individual detail of the patient's laryngeal inlet to form a satisfactory seal.

These new laryngeal airway devices with non-inflatable cuffs have proved to be a great improvement in terms of safety and reliability. They cause very much less trauma to the tissue in the patient's laryngopharyngeal framework, whilst still allowing good inflation/seal pressures.

In the case of administering anaesthetic to animals, particularly in small animals such as dogs, cats, rabbits etc, devices with an inflatable laryngeal cuff are generally neither user nor patient friendly. Problems arise because, as a rule, the epiglottis in such animals or in small human children is very much longer and floppier than the epiglottis of an adult human subject and, as such, the epiglottis tends to down fold and obstruct the outlet of the airway channel within the cuff and may interrupt or prevent the flow of anaesthetic gas. Also, because the inflatable section of an inflatable device is near the oral cavity, there is always a risk of this puncturing on the teeth during insertion, with the consequent loss of the pharyngeal/perilaryngeal seal, or at removal thus rendering an expensive reusable SGD unusable.

As a consequence of this, it is more normal to use an endotracheal tube, either inflatable or non-inflatable, when anaesthetising animals or small children. Endotracheal tubes have their own inherent problems and shortcomings, especially in the presence of insufficient local and/or sub-optimal levels of general anaesthesia.

In cats, laryngeal spasm, coughing, retching, breath holding or bronchospasm are possible during intubation. Laryngotracheal damage or rupture from wide diameter or cuffed endotracheal tubes has also been reported. Rabbits have a narrow pharyngeal inlet, a small larynx and minimal hypopharyhgeal and perilaryngeal visibility on oral examination. This makes it extremely difficult to intubate a rabbit, and normally only small endotracheal tubes can be used, if at all. It is possible that repeated blind intubation attempts may cause laryngotracheal oedema, which could then result in airway obstruction at extubation.

Endotracheal intubation necessitates the use of a short or long acting muscle relaxant as part of an anaesthetic technique to temporarily paralyse the vocal cords in order to safely and easily pass the endotracheal tube through widely open vocal cords. However, in small companion animals, due to the fear of repeated laryngoscopy attempts, or failure or loss of airway during act of intubation, muscle relaxants are very rarely used. So in the absence of muscle relaxation, the endotracheal intubation becomes even more difficult and traumatic. During insertion of endotracheal tubes in both animals and humans, the device must pass through the vocal cords and can cause damage to these cords. This damage may be permanent. If a non-inflatable tube is used then it is critical that the tube is a snug fit within the trachea. The degree of seal around the tube is heavily dependent on the anaesthetist's selection of the correct diameter tube. Intubating small animals usually requires more than one attempt to achieve successful intubation even in experienced and specially skilled hands. This can lead to trauma of the delicate pharyngolaryngeal framework that in turn can lead to swelling/oedema of the laryngeal inlet and airway obstruction on extubation which often will prove fatal in small animals. Use of excessive amounts of anaesthetic drugs to achieve favourable conditions for successful intubation, i.e. relaxed vocal cords and larynx, laryngoscopic and intubation responses, laryngo-tracheal stimulations can lead to trauma to the soft tissues, teeth or cartilages, extubation response, laryngo-bronchospasm, coughing, bleeding, dysphagia, dysphonia, sore throats and infections, patient discomfort, mucosal congestion, pharyngo-esophageal perforation, pneumothorax, subcutaneous emphysema, retropharyngeal abscess, mediastinitis, tracheo-esophageal fistula, dislocation of arytenoid/corniculate cartilages and vocal cord damage.

EP1579885 (Brain, Archibald lan Jeremy) describes a method for fabricating low cost laryngeal mask devices which have a generally elliptically shaped plate and a cuff.

EP0448878 (Brain, Archibald lan Jeremy) describes an artificial airway device to facilitate lung ventilation in an unconscious patient, comprises a mask having a flexible annular peripheral formation of roughly elliptical shape capable of conforming to, and of readily fitting within, the actual and potential space behind the larynx so as to form a seal around the circumference of the laryngeal inlet without the device penetrating into the interior of the larynx, the annular peripheral formation surrounding a hollow interior space or lumen of the mask into which opens an airway conduit whose length is of the same order as that of the mask. An airway tube is connectable to the airway conduit via a detachable connector, so that the mask can be inserted into operative position in a patient via the mouth while the airway tube can subsequently be inserted through the nose and connected to the airway conduit by the detachable connecting means.

WO01/13980 (Collins, Michael) describes a laryngeal mask airway which has a main tubular shaft with a mask portion attached at its patient end. The mask portion comprises a mount and a sealing cuff attached to the mount. The interior of the mount defines a large volume atrium communicating with the bore of the shaft at one end and with an opening through the cuff at its other end. The join between the shaft and the mount is located above the rear part of the cuff.

Another airway device is described in EP 1 675 937 (A2).

Inflatable supraglottic airway devices are commonly used in anaesthesia of humans but have hardly been used in veterinary anaesthetic practice, as those devices are designed specifically for human use.

A veterinary designed supraglottic airway system would be extremely valuable for anaesthesia, especially for small companion animals, and may also have features that would provide improvement for human use.

Accordingly, it is an object of the present invention to overcome or mitigate some or all of these problems, to provide an improved airway device for both animal and human use.

### Summary of the Invention

According to the present invention there is provided an airway device according to Claim 1. A first embodiment provides an airway device for animal or human use, said device comprising an airway tube having a distal end and a proximal end, wherein the distal end of the airway tube is surrounded by a laryngeal cuff, and wherein the face of the laryngeal cuff is shaped to form an anatomical fit over the laryngeal inlet of an animal or human patient, and to form a seal with the laryngeal inlet of the patient, wherein the face of the laryngeal cuff is a generally elongate substantially elliptical shape, the face of the cuff is substantially elliptical in shape but the shape of the opening in the face of the cuff is non-elliptical and the width of the opening at the proximal end of the face of the cuff is narrower than the width of the opening at the distal end of the airway tube wherein the face of the laryngeal cuff incorporates an epiglottic support, being so sized, shaped and positioned as to prevent the epiglottis of the patient obstructing the opening in the distal end of the airway tube in use, by retaining the epiglottis substantially clear of the opening in the end of the airway tube and wherein the airway tube has a height H and a width W and wherein the epiglottic support/rest comprises a region of the cuff or airway tube which stands above or proud of the general height H of the profile of the airway tube and the ratio A/B of the length of the major axis A of the ellipse to the length of the minor axis B of the ellipse is greater than 2.3 ± 10%.

The face of the cuff forms a substantially fluid-tight seal with the region around the laryngeal inlet of the patient in use. A substantially fluid-tight seal in this context means a seal which can contain a pressure equivalent to up to 30cm H₂O.

The distal end of the airway tube is surrounded by the laryngeal cuff and there is, inevitably, an opening in the face of the cuff in fluid connection with the airway tube. By forming the face of the cuff, and more particularly the opening in the face of the cuff, as an elongate ellipse, this enables the epiglottis to overlay part of the opening, without obstructing the airway tube.

In calculating the ratio A/B, the length of the major axis of the cuff face is taken as the distance between the distal tip of the device and the proximal extremity of the epiglottic rest, nearest to the connector end of the device. The length of the minor axis of the face of the cuff is taken as the width of the cuff at its broadest point.

In a particularly preferred embodiment the ratio A/B is greater than 2.5 ± 10%, and further preferred that the ratio A/B is 2.6 ± 10%.

The face of the cuff is substantially elliptical in shape but the shape of the opening in the face of the cuff is non-elliptical.

It will be appreciated that a laryngeal cuff is a three dimensional item. As such, it has a back and a front and a depth between the front face and the back face, although the depth may vary over the area of the cuff. The front of the cuff incorporates an opening which can be considered as an extension of the airway tube. The opening in the face of the cuff is inevitably smaller in its profile than the external profile of the cuff face itself, since the opening in the cuff fits completely within the cuff face. It will also be appreciated that the shape and profile of the opening in the cuff face may be different from the shape and profile of the face of the cuff. Thus, for example, in some of the embodiments described herein, the shape of the cuff face may be generally or substantially elliptical but the shape of the opening in the cuff face is non-elliptical.

It will also be appreciated that the cuffs of such devices must be smoothly contoured on the external regions of the cuff, in order to minimise any potential damage to sensitive tissue in the laryngopharyngeal region of the patient. It follows therefore that the front, back and sides of the laryngeal cuff will merge smoothly into one another, and the cuff itself will merge smoothly into the airway tube/buccal cavity stabilizer region. The back and front of the cuff therefore merge smoothly into each other as opposed to there being a discrete boundary between the two.

The width of the opening at the proximal end of the face of the cuff is narrower than the width of the opening at the distal end of the airway cuff.

By narrowing the opening in the face of the upper region of the cuff, being the region at the proximal end of the cuff, in the portion of the cuff where the epiglottis is likely to rest in use, then this feature significantly reduces the opportunity for the epiglottis to down fold and obstruct the opening in the cuff, and thus restrict the airway. This is particularly the case if this feature is used in combination with a raised portion at the proximal end of the cuff, and/or an elongate elliptical cuff face. This creates what is in effect a slit or elongated opening in the upper region of the cuff. This slit is approximately equal to or longer than the usual length of the epiglottis but the width of the slit is narrower than that of the epiglottis to prevent down-folding of the epiglottis into the distal opening of the airway channel, or, in the event that the epiglottis does down-fold, it is still not able to obstruct the airway.

The face of the laryngeal cuff incorporates an epiglottic support or rest, being so sized, shaped and positioned as to prevent the epiglottis of the patient obstructing the opening in the distal end of the airway tube in use, by retaining the epiglottis substantially clear of the opening in the end of the airway tube. The epiglottic support may take the form of a narrowing of the opening in the face of the cuff, or may take some other form, or both.

On the one hand the laryngeal cuff may be considered as an extension of airway tube. On the other hand, the airway tube may be considered to terminate as it enters the laryngeal cuff. In either event, it is important that the airway tube does not become blocked during use. Airway tube blockage can occur if something covers substantially all of the opening in the face of the laryngeal cuff, or if something covers or blocks the airway tube or the end of the airway tube where it enters the laryngeal cuff. The patient's epiglottis is potentially capable of causing a blockage in both these ways. An epiglottic support as described and defined herein, in one or more of its various forms, can prevent such a blockage.

The airway tube generally has a height H and a width W and the epiglottic support comprises a region of the cuff or airway tube which stands above or proud of the general height H of the profile of the airway tube.

Preferably the narrow region of the opening in the face of the cuff corresponds to the region of the epiglottic support, and may itself provide the epiglottic support.

Preferably the opening in the airway tube at the proximal end of the laryngeal cuff is substantially V-shaped, the narrow part of the V-shaped opening acting as an epiglottic rest to prevent the epiglottis of the patient obstructing the distal end of the airway tube in use_{.}

No one previously realised that by shaping the proximal end of the opening in the face of the laryngeal cuff in the shape of a "V", this could be used to keep the epiglottis should that down fold from obstructing the airway tube.

It will therefore be appreciated from the foregoing that an elongate opening in the face of an elongate laryngeal cuff will have opposing sides to the opening. Those sides will converge in some way at the proximal and distal ends of the opening. In this embodiment of the present invention the opposing sides of the opening in the proximal part of the opening create a narrow region which extends for 25% ± 10% of the length of the opening along the longitudinal axis of the device. This narrowing may be achieved by having the opposing sides meet in a V-shape but this is not the only shape which can be used to create the desired effect. A narrow U-shaped region at the proximal end of the opening would also create the desired effect.

This narrow region is generally a region whose width at the widest point of the narrow region is less than 50% of the width of the opening in the cuff face at its widest point. As such, the precise shape of this opening is not critical, although a V-shape or a narrow U-shape is convenient.

Preferably the angle formed between the two branches of the "V" is in the order of 10° to 50°, more preferably in the order of 20° to 40° and in a particularly preferred embodiment in the order of 30° ± 5°.

Also described herein the general plane of the face of the laryngeal cuff is at an angle of between 10° and 40° ± 5° to the general longitudinal axis of the airway tube, and more preferably the angle between the face of the cuff and the general longitudinal axis of the airway tube is between 15° and 25° ± 5°. Designing the face of the cuff such that there are these angles between the face of the cuff and the general longitudinal axis of the airway tube further directs the epiglottis away from the opening in the cuff face.

Preferably the face of the laryngeal cuff is formed from a material with a Shore hardness on the A scale of 40 or less, and more preferably from a material with a Shore hardness on the A scale of between 000 to 20.

In a particularly preferred embodiment the airway tube and the laryngeal cuff are formed from a material with a Shore hardness on the A scale of 40 or less, and more preferably the airway tube and the laryngeal cuff are formed from a material with a shore hardness on the A scale of 30 ± 10.

In an alternative preferred embodiment the airway tube and the laryngeal cuff are formed from the same material, being a material with a Shore hardness on the A scale of 60 or less, and more preferably 40 or less. Thus, it is the perceived softness from the patient's point of view which is important, rather than the inherent hardness/softness of the material from which the device, and in particular the face of the cuff of the device which comes into contact with the patient. This enables materials of a Shore hardness on the A scale of 40-70 to be used quite satisfactorily, whilst giving the impression that the Shore hardness is less than 40.

In a further particularly preferred embodiment, applicable to all the aspects of the present invention, the face of the laryngeal cuff, being that part of the cuff that comes into contact with the patient in use, is formed from a first material having one Shore hardness and the remainder of the body of the device is formed from a second material having a different Shore hardness. In this context, the remainder of the body of the device excludes the connector but includes the remainder or dorsal part of the laryngeal cuff, the airway tube and the buccal cavity stabiliser, if present.

Preferably face of the laryngeal cuff is formed from a first material having a Shore hardness of 20 or less on the A scale, and preferably 15 or less. More preferably the face of the laryngeal cuff is formed from a first material having a Shore hardness on the A scale of 000 to 10.

It is further preferred that the airway tube, buccal cavity stabiliser if present, and the dorsal part of the laryngeal cuff are formed from a material having a Shore hardness on the A scale of 60 or less, and preferably 50 or less. More preferably the airway tube, buccal cavity stabiliser if present, and dorsal part of the laryngeal cuff are formed from a material having a Shore hardness on the A scale in the range 20 to 40.

It will also be appreciated that both the cuff and the outer part of the airway tube which comes into contact with the patient can be formed from plastics material, preferably a thermoplastic material and/or a cross-linked (thermoset) material, including electrometric rubber materials, having substantially the same Shore hardness. A connector is required to connect the device to a supply of anaesthetic gas. This connector can extend into the airway tube, as a tube liner and bite protector. The connector can thus act to increase the rigidity of the device, where the connector is formed from a non-collapsible relatively hard material. The connector can be integrated in such a way that it not only will act as a connector but also provides internal support to the device to give it the desired rigidity as well as acting as a bite block. In addition, the external surface of the airway tube may incorporate external ridges. These ridges, which in a preferred embodiment are at or near the proximal end of the device, assist in tying or taping the device to the patient, particularly an animal patient where the animal's face is covered in hair or fur. The ridges can be located at any suitable location along the outside of the airway tube.

In a particularly preferred embodiment the whole of the device, with the exception of the connector portion, is formed from material of substantially the same Shore hardness on the A scale. A preferred Shore hardness is 60 or less and a particularly preferred Shore hardness is 40 or less. The perceived softness of the device can be modified by incorporating channels or cavities within the body of the device, for example a honeycomb structure, in addition to the airway tube. Alternatively the thickness of various sections can be reduced. In both these circumstances the airway device will feel softer than the Shore hardness of the material of construction might suggest. Thus, it is the perceived softness from the patient's point of view which is important, rather than the inherent hardness/softness of the material from which the device, and in particular the face of the cuff of the device which comes into contact with the patient. This enables materials of a Shore hardness on the A scale of 40-70 to be used quite satisfactorily, whilst giving the impression that the Shore hardness is less than 40.

Also described herein the airway device further comprises a buccal cavity stabilizer, said buccal cavity stabiliser being adapted to rest on the anterior aspect or front of the patient's tongue, more so towards the base of the tongue and being configured to correspond with the anatomy of that part of the patient, and thus provide stability to prevent rotational or side-to-side movement of the airway tube in use.

Preferably the softness of the laryngeal cuff is adapted by forming cavities or channels within the body of the cuff itself. This enables the device to be formed of a harder, firmer plastics material and yet still have the desired softness in the cuff region.

Preferably the face of the laryngeal cuff and a front, ventral part of the buccal cavity stabiliser are formed from a material of substantially the same Shore hardness. This simplifies construction and ensures that all portions that come into contact with the patient's soft tissue are relatively soft.

Preferably the buccal cavity stabiliser has a first, ventral face in substantially the same plane as the plane of the opening in the laryngeal cuff.

Preferably the buccal cavity stabiliser extends from the proximal end of the laryngeal cuff towards the proximal end of the airway tube such that the cuff and the buccal cavity stabiliser are of integral construction, and preferably the laryngeal cuff, epiglottic support, buccal cavity stabilizer (if present) and the airway tube are formed during manufacture as an integral unit.

Preferably the buccal cavity stabiliser is non-uniform in its width W, having a wide point located at a point intermediate the laryngeal cuff and the proximal end of the airway tube, and preferably the wide point of the buccal cavity stabiliser is closer to the laryngeal cuff than to the proximal end of the airway tube.

Preferably the ratio of the width W of the buccal cavity stabiliser at its widest point to the height H of the buccal cavity stabiliser at that same point is between 2 and 3 ± 20% and more particularly 2.5 to 2.7 ± 20%.

Preferably the face of the buccal cavity stabiliser which comes into contact with the patient's tongue is roughened to increase friction of the stabiliser with the tongue in use.

Preferably the buccal cavity stabiliser is formed as an integral part of the airway tube.

Preferably the buccal cavity stabilizer is non-inflatable.

Also described herein the device further incorporates a gastric tube passageway extending from the tip of the cuff to the proximal end of the device, and preferably the gastric tube passageway is housed within the body of the device.

Preferably the gastric tube passageway extends alongside the airway tube.

Preferably the gastric tube passageway runs from an opening in the proximal end of the device to an opening in a distal tip of the laryngeal cuff.

Preferably the epiglottic rest further comprises a leaf-like structure extending away from the laryngeal cuff and back towards the proximal end of the device (connector end).

Preferably the laryngeal cuff further comprises one or more featherlike flanges. These flanges improve the quality of the seal between the face of the device and the patient's laryngeal inlet.

Also described herein the distal tip of the device is so sized and shaped as to form an oesophageal obturator, blocking in use the opening into the subject's oesophagus by forming a substantially fluid tight seal therein. By lengthening the length of the A or major axis of the substantially elliptical shaped cuff, the distal tip of the cuff can be designed to reach to and partially into the subject's oesophagus.

The volume of the distal tip can also be designed to be of a shape which will block the subject's oesophagus in use.

Thus the distal tip of the device is preferably formed as a broad extension to the generally elongate substantially elliptical face of the laryngeal cuff. This is one reason why the shape of the laryngeal cuff may deviate from a true ellipse.

Preferably the dorsal or posterior face of the laryngeal cuff incorporates one or more protuberances, said protuberances being sized and shaped such that they contact, in use, the back of the pharynx of the subject. By including a raised area on the back of the cuff which makes contact, in use, with the structure opposite the subject's laryngeal inlet, this creates a gentle force which acts to hold the device against the laryngeal inlet and hence improves the seal between the two.

Preferably the protuberances take the form of a soft raised cushion. The raised region is preferably behind the proximal part of the opening in the cuff face.

In a further preferred embodiment the front, ventral part of the cuff may be inflatable. Inflatable laryngeal cuffs are known per se, but not with the specific shapes and profiles described herein. An inflatable cuff on the front face of the laryngeal cuff can be used with or without an inflatable dorsal cuff or cushion. The choice will be made in part depending on which animal the particular device is intended to be used on.

Also described herein there is provided an airway device for animal or human use, said device comprising an airway tube having a distal end and a proximal end, wherein the distal end of the airway tube is surrounded by a laryngeal cuff, and wherein the face of the laryngeal cuff is shaped to form an anatomical fit over the laryngeal inlet of an animal or human patient, and to form a substantially fluid-tight seal with the laryngeal inlet of the patient, wherein the face of the cuff is a generally elongate substantially elliptical shape, the face of the laryngeal cuff incorporating an opening, wherein the opening in the laryngeal cuff tapers or narrows towards the proximal end of the cuff.

Preferably the opening in the airway tube at the proximal end of the laryngeal cuff is substantially V-shaped, the narrowing of the V-shaped opening acting as an epiglottic rest to prevent the epiglottis of the patient obstructing the distal end of the airway tube in use.

Preferably the ratio A/B of the length of the major axis A of the ellipse to the length of the minor axis of the ellipse B is greater than 2 ± 10%, more preferably greater than 2.3 ± 10%, and even more preferably greater than 2.5 ± 10%.

In a particularly preferred example the ratio A/B is 2.6 ± 10%.

Preferably the face of the cuff is substantially elliptical in shape but the shape of the opening in the face of the cuff is non-elliptical.

Preferably the width of the opening at the proximal end of the face of the cuff is narrower than the width of the opening at the distal end of the airway cuff.

Preferably the face of the laryngeal cuff incorporates an epiglottic rest, being so sized, shaped and positioned as to prevent the epiglottis down folding of the patient obstructing the opening in the distal end of the airway tube in use, by retaining the epiglottis substantially clear of the opening in the face of the laryngeal cuff.

Preferably the airway tube has a height H and a width W and wherein the epiglottic support comprises a region of the cuff or airway tube which stands above or proud of the general height H of the profile of the airway tube.

Preferably the narrow region of the opening in the face of the cuff corresponds to the region of the epiglottic rest. In one example the narrowed region in the opening in the cuff face acts as the epiglottic rest.

Preferably the general plane of the face of the laryngeal cuff is at an angle of between 10° and 40° + 5° to the general longitudinal axis of the airway tube, and more preferably the angle between the face of the cuff and the general longitudinal axis of the airway tube is between 15° and 25° ± 5°.

Preferably the face of the laryngeal cuff is formed from a material with a Shore hardness on the A scale of 60 or less, preferably 40 or less, and more preferably the face of the laryngeal cuff is formed from a material with a Shore hardness on the A scale of between 000 to 20. It will be appreciated that, in use, only the face of the cuff comes into direct contact with the laryngeal inlet of the patient. Thus the face of the cuff may be formed from a different, generally softer, material than the remainder of the body of the cuff. Where different materials are used, this can be in the form of an overlap or overlay of softer material.

In a particularly preferred example the airway tube and the laryngeal cuff are formed from a material with a Shore hardness on the A scale of 60 or less, and more preferably the airway tube and the laryngeal cuff are formed from a material with a shore hardness on the A scale of 40 or less, more preferably 30 ± 10.

In an alternative preferred example the airway tube and the laryngeal cuff are formed from a material with a Shore hardness on the A scale of 20 or less.

Preferably the device further comprises a buccal cavity stabilizer, said buccal cavity stabiliser being adapted to rest on the anterior aspect or front of the patient's tongue and being configured to correspond with the anatomy of that part of the patient, and thus provide stability to prevent rotational or side-to-side movement of the airway tube in use.

Preferably the softness of the laryngeal cuff is adapted by forming cavities or channels within the body of the cuff itself.

Preferably the face of the laryngeal cuff and a front, ventral part of the buccal cavity stabiliser are formed from a material of substantially the same Shore hardness.

Preferably the buccal cavity stabiliser has a first, ventral face in substantially the same plane as the plane of the airway tube.

Preferably the buccal cavity stabiliser extends from the proximal end of the laryngeal cuff towards the proximal end of the airway tube such that the cuff and the buccal cavity stabiliser are of integral construction.

Preferably the buccal cavity stabiliser is non-uniform in its width W, having a wide point located at a point intermediate the laryngeal cuff and the proximal end of the airway tube, and preferably the wide point of the buccal cavity stabiliser is closer to the laryngeal cuff than to the proximal end of the airway tube.

Preferably the ratio of the width W of the buccal cavity stabiliser at its widest point to the height H of the buccal cavity stabiliser at that same point is 2.7 ± 20%.

Preferably the face of the buccal cavity stabiliser which comes into contact with the patient's tongue is roughened to increase friction of the stabiliser with the tongue in use.

Preferably the buccal cavity stabiliser is formed as an integral part of the airway tube, and more preferably the buccal cavity stabiliser, the airway tube and the laryngeal cuff region are formed as an integral unit.

Preferably the buccal cavity stabilizer is non-inflatable.

Also described herein the buccal cavity stabilizer is inflatable.

Preferably the device further incorporates a gastric tube passageway extending from the lip of the cuff to the proximal end of the device, and preferably the gastric tube passageway is housed within the body of the device.

Preferably the gastric tube passageway extends alongside the airway tube, and more preferably the gastric tube passageway runs from an opening in the proximal end of the device to an opening in a distal tip of the laryngeal cuff.

Preferably the epiglottic support further comprises a leaf-like structure extending away from the laryngeal cuff.

Preferably the face of the laryngeal cuff further comprises one or more featherlike flanges.

Preferably the distal tip of the device is so sized and shaped as to form an oesophageal obturator, blocking in use the opening into the subject's oesophagus by forming a substantially fluid tight seal therein. By lengthening the length of the A or major axis of the substantially elliptical shaped cuff, the distal tip of the cuff can be designed to reach to and partially into the subject's oesophagus. The volume of the distal tip can also be designed to be of a shape which blocks the subject's oesophagus in use.

Thus the distal tip of the device is preferably formed as a broad extension to the generally elongate substantially elliptical face of the laryngeal cuff. This is one reason why the shape of the laryngeal cuff may deviate from a true ellipse

Preferably the dorsal or posterior face of the laryngeal cuff incorporates one or more protuberances, said protuberances being sized and shaped such that they contact, in use, the back of the pharynx of the subject. By including a raised area on the back of the cuff which makes contact, in use, with the structure opposite the subject's laryngeal inlet, this creates a gentile force which acts to hold the device against the laryngeal inlet and hence improves the seal between the two.

Preferably the protuberances take the form of a soft raised cushion. The raised region is preferably behind the proximal part of the opening in the cuff face.

In a further preferred example the front, ventral part of the cuff may be inflatable. Inflatable laryngeal cuffs are known per se, but not with the specific shapes and profiles described herein. An inflatable cuff on the front face of the laryngeal cuff can be used with or without an inflatable dorsal cuff or cushion. The choice will be made in part depending on which animal the particular device is intended to be used on.

The present invention also extends to methods of manufacturing an airway device of the type claimed herein.

Manufacturing methods include moulding an airway tube, laryngeal cuff and epiglottic support and, if present, a buccal cavity stabiliser, around a connector adapted to connect to an anaesthetic gas delivery unit.

A two shot injection moulding process can be used in the event that different parts of the device are formed from different plastics material, or plastics materials of different Shore hardness. In addition, insert-moulding techniques can be used. In the case of thermoset cross-linking electrometric materials, moulding techniques such as compression moulding can be used. Liquid silicone rubber and solid silicone rubber materials require special moulding techniques, as will be determined by the materials specialist, depending on the particular plastics materials being used.

Alternatively, an inner airway tube and connector may be formed in a first step, and the remainder of the device moulded around that component in a second step.

In a further alternative an inner airway tube and connector may be formed in a first step with a sleeve of the same Shore hardness extending up to the back plate of the cuff providing support to the airway channel and part of the cuff as a backbone/spine, the remainder of the device being moulded around that component in a second step using a soft material having a Shore hardness of 000 to 20 ± 5.

In a still further process the laryngeal cuff, epiglottic support, and the outer part of the airway tube may be moulded as a single component, incorporating a buccal cavity stabiliser if present. This component can then be threaded over a pre-formed connector component, which may or may not include an inner airway tube/bite protector.

Also described herein there is provided a method of anaesthetising an animal or human subject with a laryngeal device as claimed herein comprising the steps of:-
(i) providing a laryngeal airway device as claimed herein;
(ii) inserting said device into the subject such that it forms a substantially fluid-tight and airtight seal with the laryngeal inlet of the subject;
(iii) administering anaesthetic gas to the subject through the device.

### Brief Description of the Drawings

The invention will now be described, by way of example only, with reference to the accompanying drawings in which:-
Figures 1 and 2 illustrate disconnected front and back axonometric views respectively of a first embodiment of the present invention;
Figures 3 and 4 illustrate front and back axonometric views respectively of the embodiment shown in Figures 1 and 2 in assembled form;
Figures 5 to 9 illustrate back, front, side, cross-sectional and end views respectively of the assembled embodiment shown in Figures 3 and 4;
Figures 10 to 15 illustrate various views of a further embodiment, without a connector, which incorporates ridges along the airway tube and an inflatable cuff;
Figures 16 to 20 illustrate various views of a further embodiment;
Figures 21 to 24 illustrate various views of an embodiment including a gastric tube passageway;
Figure 25 illustrates a perspective view of a further embodiment suitable for larger animals;
Figure 26 illustrates an exploded view of an inflatable cuff assembly on the dorsal face of the device shown in Figure 25;

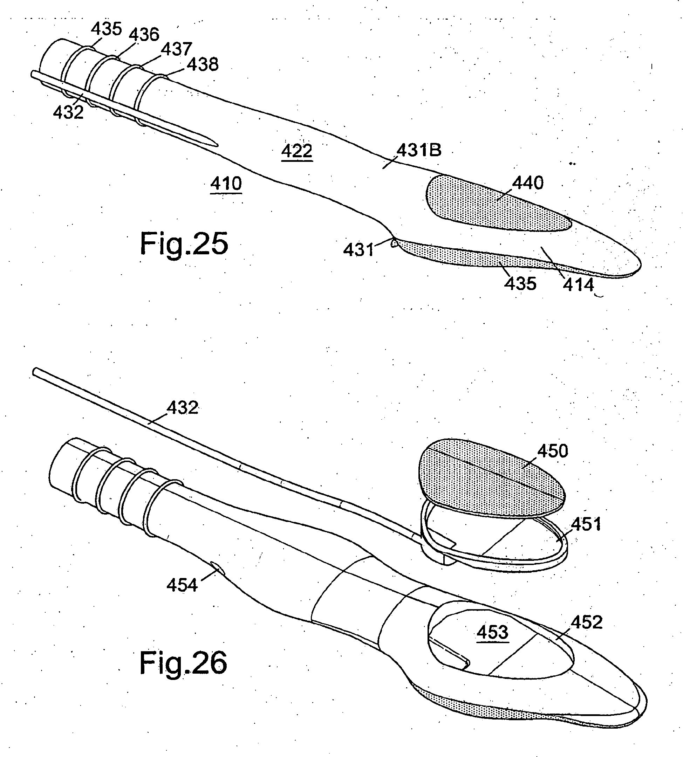

Figures 26, 27, 28 and 32 show back, front, side and end views respectively of the device of Figure 25;

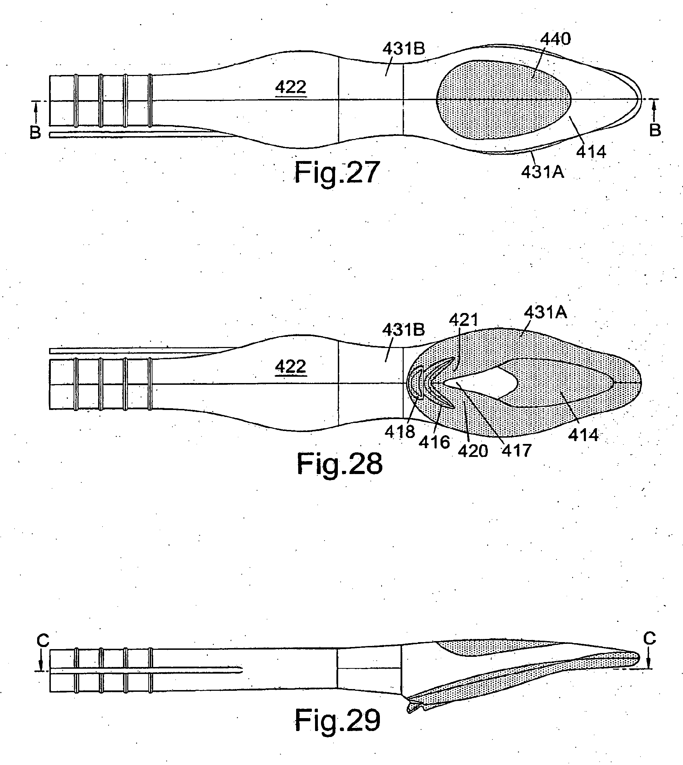

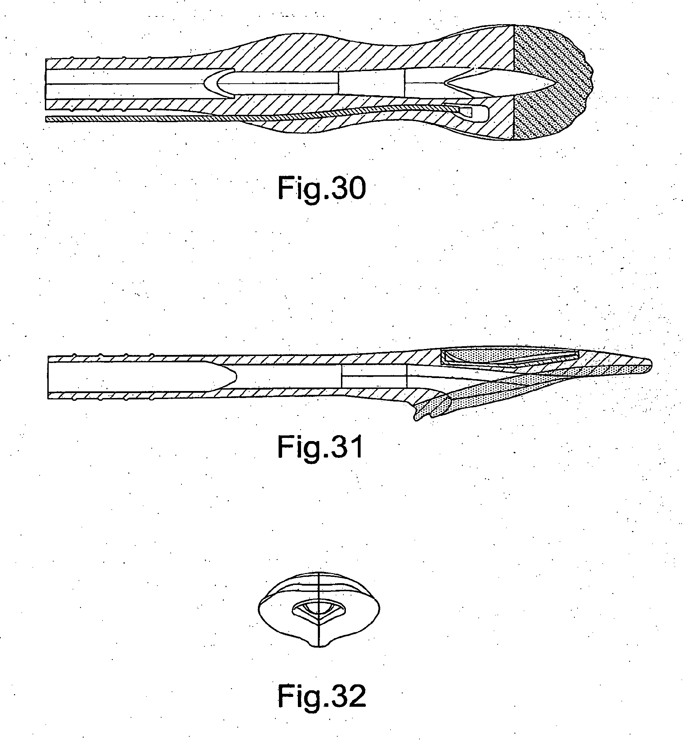

Figure 30 illustrates a sectional view along line C-C in Figure 29;
Figure 31 illustrates a sectional view along line B-B in Figure 27.

### Description of the Preferred Embodiments

Embodiments of the present invention are described below by way of example only. These examples represent the best ways of putting the invention into practice that are currently known to the applicant although they are not the only ways in which this could be achieved.

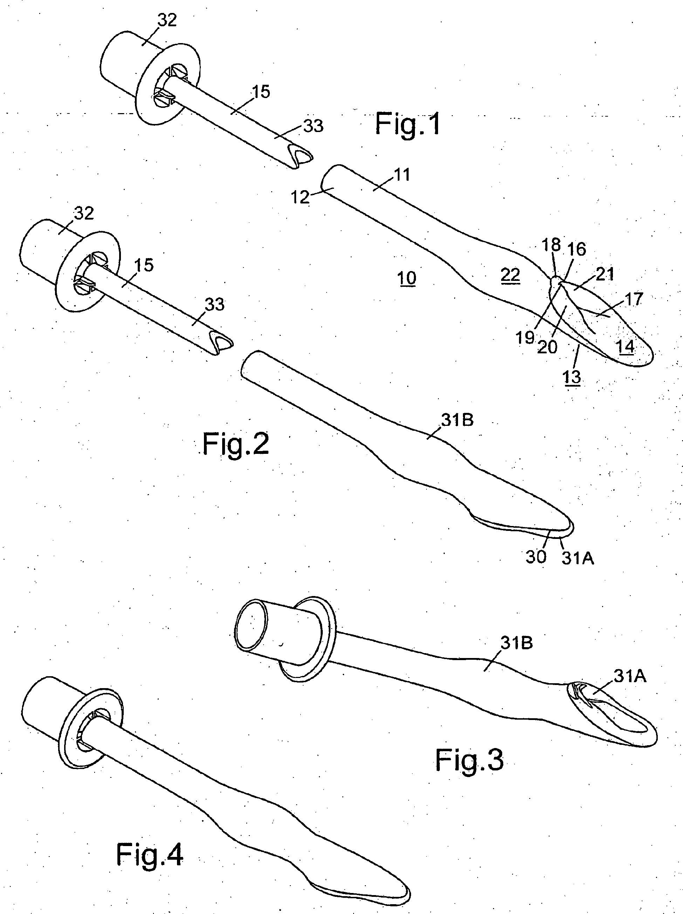

Referring to Figure 1, this shows a laryngeal mask airway device 10 according to a first embodiment of the present invention in a disassembled state. This comprises an airway tube 11, which at its proximal end 12 terminates in a 15mm or other connector 15 suitable for connection to an anaesthetic breathing system of conventional type. Formed around the distal end 13 of the airway tube is a laryngeal cuff or cup 14 adapted in its shape and contours to correspond with the larynx inlet region of a patient. In this context the terms cuff and cup have an equivalent meaning. They refer to the element of the device at the distal end of the airway tube that is adapted to cover and form a seal with the laryngeal inlet of the patient when the device is in use.

It will be seen from Figures 1 to 9 that the laryngeal cuff merges smoothly into the body of the airway tube. Because of the smooth transition between the cuff and the airway tube there is no discrete boundary between the cuff region and the airway tube in this embodiment. The term "cuff region" is therefore defined as that part of the device beyond the buccal cavity stabiliser region 22 towards the distal end of the device. It will also be understood that a ventral portion of the cuff region incorporates a cuff face. The face of the cuff is the region which interacts, in use, with the subject's laryngeal inlet. The characteristics of the face of the cuff are described in more detail below.

The cuff region comprises a front, ventral portion and a rear, dorsal portion. It will be appreciated that there is generally no discrete boundary between these two portions, especially when both the ventral and dorsal portions are formed from the same material ie from a material of the same Shore hardness. On occasions, as for example item 30 in Figure 2, a demarcation between the ventral and dorsal portions can be seen. This demarcation is particularly noticeable when the front face of the laryngeal cuff 31A is formed from a first material of different Shore hardness to the remainder of the cuff and the airway tube and/or buccal cavity stabiliser 31 B. Typically the face of the laryngeal cuff 31A is formed from a first, softer material than the remainder of the cuff/airway tube 31 B, which is formed from a second material.

The regions of different Shore hardness can also be seen in Figure 3.

By way of typical Shore hardnesses, the first material making up the face of the cuff is typically a Shore hardness of 20 or less on the A scale, and more preferably 15 or less. A particularly preferred range of Shore hardness is 000 to 10 inclusive on the A scale. It will be understood that 000, or triple zero, includes materials whose hardness, or more particularly softness, is too soft to measure on the A scale.

The second, harder material, making up the back or dorsal part of the cuff and the airway tube is typically formed from a material having a Shore hardness on the A scale of 60 or less, and preferably 50 or less. A particularly preferred hardness range is 20 to 40.

Plastic items made up of plastics with two different hardnesses are known, as is the two shot injection process for their manufacture.

The ventral portion includes the face of the cuff and also the airway opening which connects with the airway tube. It is the shape of the cuff face in these various embodiments which imparts some of the important properties to airway devices according to the present invention. In the context of this description, the term proximal means the end of the device, or portion thereof, closest to the connection to the anaesthetic breathing system. The term "distal" means the end of the device, cr portion thereof, furthest from the anaesthetic breathing system in use.

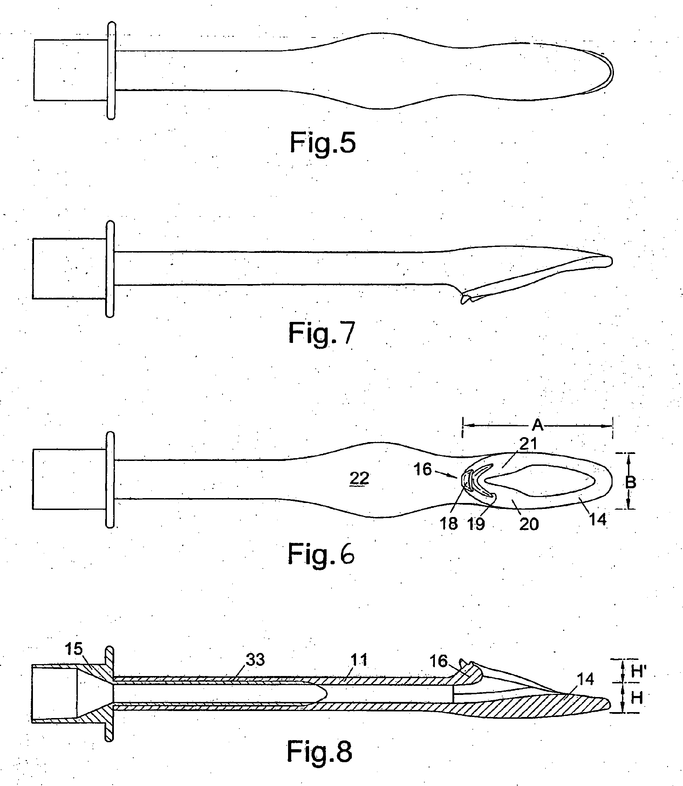

The laryngeal cuff 14 is non-inflatable in this example. Non-inflatable cuffs are known per se, for example from GB2,383,399 (Nasir). This particular cuff incorporates features designed to prevent the epiglottis of the animal or human subject from obstructing the airway opening 17 in the face of the laryngeal cuff. The first of these features is an epiglottic support 16. This structure, located at the proximal end of the ventral portion of the cuff, stands above and well proud of the general profile of the airway tube. This can be seen more clearly from the cross-section in Figure 8, where the general height of the airway tube, as it extends from the connector 15 until it begins to merge into the cuff, is shown as H. The height of the epiglottic support above this general height H is shown as H¹. Thus the epiglottic support may extend proud of the general longitudinal profile of the airway tube by an amount H ± 100%H, preferably H ± 50%H, and more preferably H ± 25%H. This represents a protrusion which is significantly larger in height than is to be found in any previous non-inflatable cuffed airway devices. Thus H' preferably equals H ± 100% H and more preferably H' equals H ± 25%H.

Considering the height of the epiglottic support in another way, the total height of the device in the epiglottic support region is defined as H + H¹. When H + H¹ is expressed as a ratio over H, the general height of the airway tube, then the ratio (H + H¹)/H equals between 1.5 and 3 in the preferred embodiments, more preferably between 1.7 and 2.5.

The epiglottic support 16 merges into the cuff face as it extends towards the distal tip of the device. This can be seen from Figures 1, 3 and 6.

The structure of the epiglottic support may take a number of forms and may include a number of features. It may, for example, include a feather shaped epiglottic lip or flange 18. This lip extends upwards and backwards towards the proximal end of the device, and away from the open face of the cuff. This lip is sized and shaped so as to be anatomically positioned against the epiglottis, to ensure a proper seal and to hold the epiglottis back from folding towards the laryngeal inlet thus avoiding obstruction to airflow. This lip takes the form of a leaf-like structure extending out of the laryngeal cuff and directed back towards the proximal end of the airway tube. Its relative size and shape can be seen from Figures 1, 3, 7 and 8. The optimum size and shape will be determined by experimentation, and may depend on the animal species for which the airway device is intended.

Whilst this lip is shown in this example as an upstanding leaf-like structure, it will be appreciated that other shapes could have the same desired effect, of retaining, in use, the epiglottis of the patient clear of the airway opening in the face of the cuff.

Figure 1 also shows a further feature of the design, namely a thin, flexible feather-like flange 19 which extends around the proximal part of the cuff opening.

The flange 19, shown more clearly in Figure 6, is preferably formed as an integral part of the moulding of the cuff and, because of the very soft nature of the material used to form the cuff, this flange is particularly soft and pliable. Its purpose is to make allowance for any individual patient variation in the laryngeal inlet shape and to contribute to forming an efficient and effective seal between the cuff and the laryngeal structures. By this design, and by designing the cuff to be a close anatomical fit, the obtainable seal pressure from experimental trials is well in excess of 30cm H₂O.

Whilst only one flange is shown in Figure 1, it will be appreciated that more than one flange could be provided. Such multiple flanges could be arranged in a concentric fashion, spaced radially apart from each other. Furthermore, the extend to which these flange(s) extend around the circumference of the laryngeal cuff can be varied, and need not be exactly as shown In Figure 1.

A further feature designed to prevent the epiglottis of the subject from obstructing the airway opening in the face of the laryngeal cuff is the raised, narrowed region at the proximal end of the cuff face shown as 20 and 21 in Figure 1. The face of a conventional laryngeal cuff, be it inflatable or non-inflatable, is that of a broad ellipse, tapering slightly towards the distal lip of the cuff. In this invention an entirely new shape for the face of the laryngeal cuff has been created. The outer profile of the face of the cuff itself is substantially that of an elongate ellipse. If the length of the major axis of the ellipse is defined as A, and the length of the minor axis of the ellipse is defined as B, as illustrated in Figure 6, then the ratio of A:B is greater than 2 ± 10%. The ratio of A:B is preferably greater than 2.3 ± 10% and more preferably about 2.5 ± 10%. In a particularly preferred embodiment the ratio of A:B is 2.6 ± 10%.

However, the shape of the opening within this elongate ellipse cuff face is not uniform along the major axis A. The size of the opening tapers or narrows towards the proximal end of cuff, such that in the region of the epiglottic support the opening is V-shaped. In this embodiment this narrowing is created by two, opposing bulges 20 and 21 and these bulges are also substantially raised above the general profile of the airway tube. The opening in this narrowed region is too narrow to allow the subject's epiglottis to fall into the airway tube opening and this narrowing, in combination with the raised epiglottic support, ensures that the airway tube remains open at all times. Whilst the subject's epiglottis may lay down folded partway over the face of the laryngeal cuff in use, the elongate shape of the face of the cuff, the narrowed opening in the face of the cuff towards its proximal end, and the raised epiglottic support, all serve to keep the airway clear and from obstruction by the patient's epiglottis.

The angle formed between the two legs of the "V" in the example shown in Figures 1 to 8 is in the order of 30° ± 5°. But this angle is not the only angle that can be used. Suitable angles include angles between 50° + 5° to 10° + 3°.

It will also be appreciated that the internal profiles of the bulges 20,21 forming this so-called "V" shape need not be linear. Thus the legs of any "V" need not be in a straight line. For example, the V-shape may tend to splay outwards as it extends towards the distal tip of the cuff.

In addition, it will be appreciated that narrowing of the opening in the cuff face towards the proximal end of the cuff which acts as an epiglottic support need not come to a discrete point, as at the bottom of a conventional "V" shape. The shape of the opening at the proximal end or base of the cuff could be curved or even flat or planar. What is important is that the opening in that region is narrow enough to prevent the patient's epiglottis falling into the cavity within the cuff and obscuring the end of the airway tube. This manifests itself by a region of up to 50% of the length of the opening in the cuff face having an opening width which is less than 50% of the width of the opening in the cuff face at its widest point. Preferred parameters are a length of 30% of the length of the opening in the cuff face having an opening width which is less than 40% of the width of the opening in the cuff face at its widest point.

It will be understood that the narrowing of the feature of a V-shaped opening in the face of the laryngeal cuff can be used independently of other epiglottic support features. Alternatively, the feature of a V-shaped opening can be omitted altogether, in which case the epiglottic support will take a different form.

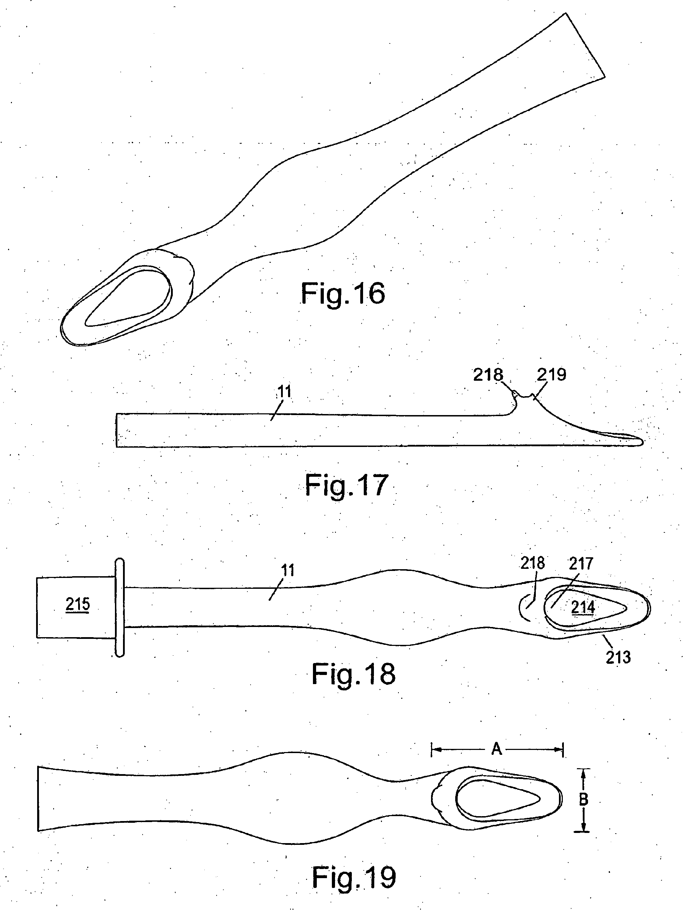

It will be appreciated that, while all the features described above are advantageous, they can be incorporated into an airway device separately and independently as selected by the designer, and are not all required to be present in order to create the desired effect and are thus not all essential. Nor does one feature depend on another for their effect. So, for example, a narrow, V-shaped opening at the proximal end of the cuff face opening can be used without a raised epiglottic support if desired. And, as will be shown in Figures 16 to 24 an elongate ellipse-shaped cuff can be used without a V-shaped opening.

The airway device shown in the Figures also includes a buccal cavity stabiliser 22,122. Such stabilisers, also known as pharyngeal stabilisers, are described in detail in GB2,393,399 the text of which is hereby imported by reference and is intended to be an integral part of this disclosure. In this example this stabiliser takes the form of an expanded region extending symmetrically on either side of the airway tube. This stabiliser is adapted to rest on the anterior aspect or front of the tongue and is configured to correspond with the anatomy of that part of the patient.

A wide variety of shapes, sizes, and positions for this stabiliser are possible. The face of the stabiliser may be roughened, scored or serrated to increase the friction with the tongue and thus stabilise it in use, to avoid or reduce forward or backwards movements when in use.

In this example the stabiliser is formed integrally with the cuff, such that one runs smoothly into the other. However, this is not essential and the stabiliser could be a separate unit on the airway tube. An essential feature of the stabiliser is that it is a region which is broader in cross-section than the diameter of the airway tube itself. That is to say it extends into a region either side of the airway tube and in the same generally plane as the laryngeal cuff.

A stabiliser could be formed from any, preferably soft, extension around the airway tube wall that can lie on the anterior aspect of the tongue. Any suitably shaped laterally extending flange whose body lies mainly along the longitudinal axis of the airway tube would serve this purpose. The flange need not be solid, so a tubular, mesh or other perforated structure would be perfectly acceptable.

It is also possible to form such a "flange" by broadening the profile of the airway tube at the appropriate region, just above the cuff. That is to say, the buccal cavity stabiliser may be an integral part of the airway tube, rather than being a separate component formed on or around the airway tube itself. Thus, a suitable increase in the general profile of the airway tube, whether formed by the tube itself or by forming additional material around the tube, can act as a stabiliser.

The general profile of the buccal cavity stabiliser and the way that it merges smoothly into the cuff region can be seen in more detail from Figures 5 and 6.

It will be appreciated that whilst a buccal cavity stabiliser is a preferred feature in such an airway device, it is not an essential feature.

The connector 15 in this example consists of a connector proximal portion 32, designed to connect onto an anaesthetic gas delivery device, and a connector distal tubular portion 33, which fits snugly inside the airway tube 11. Portion 33 also acts as a bite protector, preventing the patient's teeth from puncturing the airway tube. Portion 33 also prevents occlusion of the airway in the event that the subject should bite down on the airway tube.

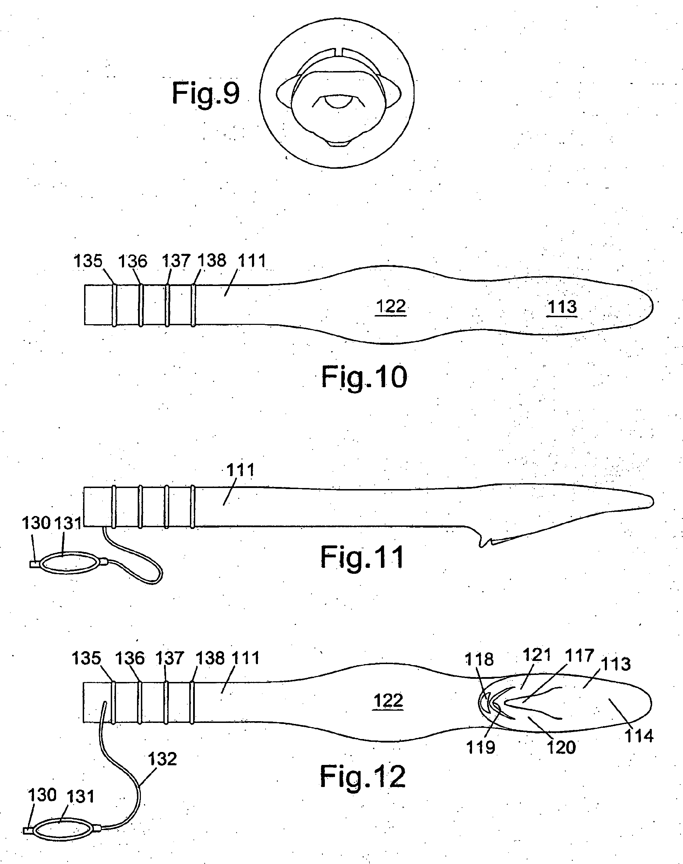

Figures 10 to 15 inclusive illustrate the airway tube and cuff, but not the connector, of a further embodiment in which the cuff is inflatable. This embodiment also includes a series of ridges on the external surface of the airway tube at or near the proximal end of the tube and which are described in more detail below.

An inflation tube 132 is provided and this preferably runs internally of the device for part of its length within or alongside the airway tube and into an inflatable region of the cuff 113. The proximal end of the inflation tube 132 includes a one-way valve fitting 130 suitable for connection to a syringe body. This allows air to be introduced into the inflatable cuff via the syringe. A small balloon 131 in the inflation tube gives a visual indication to the operator of the degree of inflation of the cuff.

The technology for producing inflatable cuffs is well known. An inflatable region within the cuff is formed during the moulding or manufacturing process, and this is connected to the inflation tube 132.

Any suitable connector, such as that shown as 15 in Figure 1 can be inserted into the end of the airway tube 111 to complete the airway device.

A series of ridges 135,136,137 and 138 are provided, the ridges being spaced one from another along the outside of the airway tube, along the longitudinal axis, at the proximal end of the device. Since most animals are covered in hair, this prevents or complicates an airway device being taped securely, in use, to the animals' head. Instead, these devices may be tied to the animal's head and these ridges assist in this tying process.

A feature of the interconnection between the connector and the body of the device is shown in Figure 8. In this example the connector 15 also incorporates a portion of the airway tube 33. This fits inside the softer airway tube 11 of the device and, in doing so, provides some additional rigidity where the airway device is formed from a very soft plastics material.

In terms of preferred materials, in one preferred embodiment the cuff is non-inflatable and is formed from any suitable soft plastics material. By way of a preferred softness (hardness) range, on the Shore A scale of Hardness, a hardness of less than 60 for the face of the cuff that contacts the laryngeal inlet is optimum.

By way of a preferred range, a value on the same scale of between 000 to 40 is preferred, with a particularly preferred range of 000 to 12. The softness of the cuff can be further adapted by forming cavities or channels within the body of the cuff itself.

Referring to the cross-section shown in Figure 8, it will be seen that the connector 15 and associated tube 33 are formed from one plastics material and the remainder of the device is formed from another, softer plastics material of one of the Shore hardnesses described above. A preferred range of Shore hardnesses for the connector portion is in the order of 80 on the A scale. Where a bite protector is included the Shore hardness may be greater than 80 in order to achieve the necessary strength and rigidity.

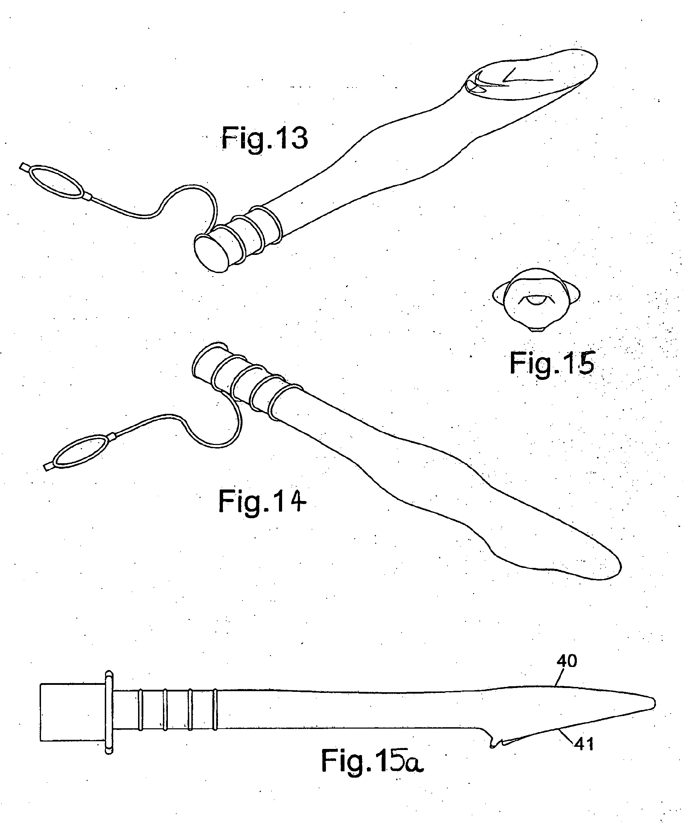

In the embodiments so far described the profile of the face of the cuff when viewed from the side, such as in Figures 7, 8 and 11, is not totally flat. Slight indentations and protuberances are provided to better fit with the anatomy of the patient's laryngeal inlet. However, it has unexpectedly been found that these indentations are not essential and acceptable sealing pressures can be obtained with devices with a substantially planar cuff face, such as shown in Figure 15A. In this embodiment the face of the cuff 41 is substantially planar. The rear or dorsal part of the cuff incorporates a protrusion or raised area 40 on the back of the cuff which contacts in use, the structure opposite the subject's laryngeal inlet, as described above. This feature results in an improved seal between the airway device and the laryngeal inlet of the subject. The protrusion may be larger than that shown in Figure 15A and will depend, in part, on the anatomy of the animal subject for which the airway device is designed. The protrusion may also be formed by an inflatable region on the dorsal part of the cuff as illustrated in Figures 25 to 32 and as described below.

The effectiveness of these planar-faced devices may be enhanced by incorporating a bulge or protuberance on the rear or dorsal portion of the cuff. Such an outbulging presses against the back of the pharynx in use and improves the sealing contact between the cuff face and the laryngeal inlet.

Further embodiments are shown in Figures 16 to 24 inclusive. These further embodiments all share the feature of having a pronounced, raised epiglottic support which stands well proud of the general profile of the airway tube when viewed in side elevation. As a result of this raised epiglottic rest, the face of the laryngeal cuff is both angled with respect to the longitudinal axis of the airway tube and tends to be slightly concave in the profile of the face of the cuff when viewed in side elevation as shown, for example, in Figure 17.

Another feature shared by these embodiments is that ratio A/B of the length of the major axis A of the ellipse of the cuff face to length of the minor axis B of the ellipse is at least in the order of 2.0. The method of calculating the ratio A/B is as described above.

A similar numbering scheme is used in these figures to that used in Figures 1 to 8.

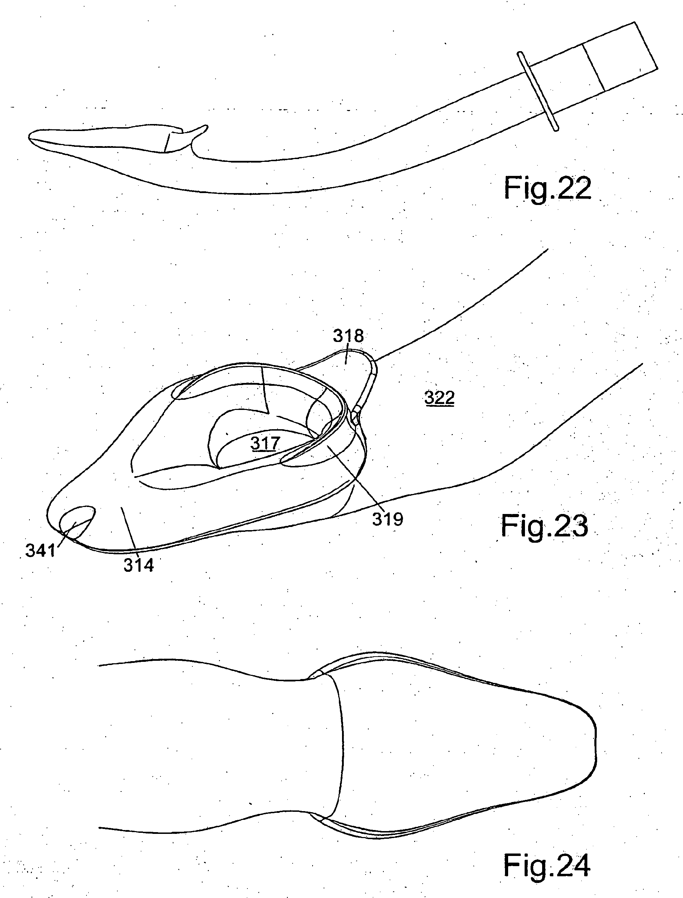

As well as a stretched elliptical or substantially elliptical cuff face, these embodiments include a highly pronounced epiglottic support, as shown in Figures 17 and 22.

The distal tip of the cuff 214 is stretched and more elongate than in known airway devices. This enables the tip of the cuff to extend into the subject's epiglottis in use and to block the epiglottis and to form a substantially fluid-tight seal therein.

The combination of the elongate elliptical shape of the cuff face and the raised epiglottic support mean that a V-shaped opening in the face of the cuff is no longer a necessity for the device to work effectively.

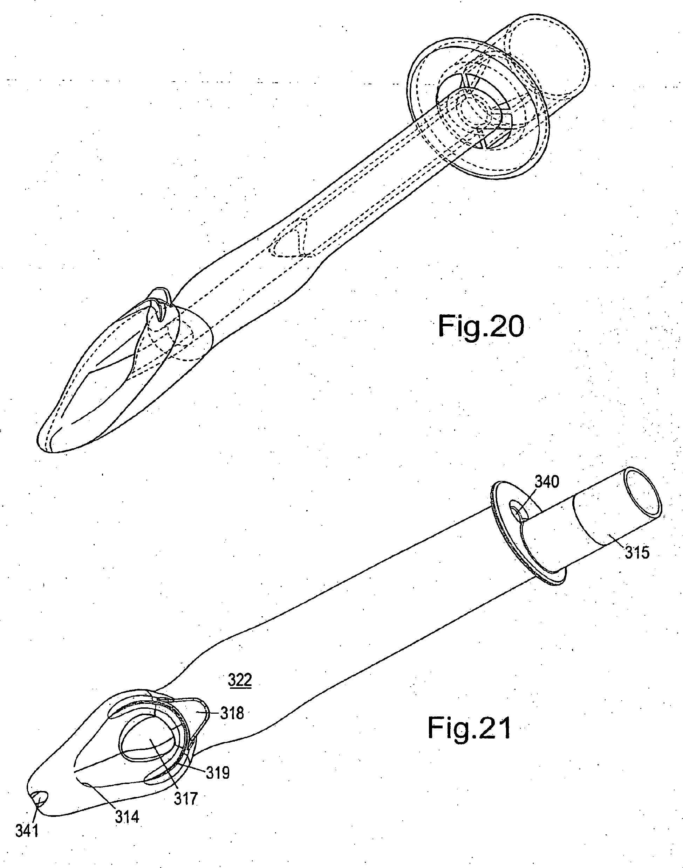

The embodiment illustrated in Figures 21 to 24 incorporates a gastric tube passageway. Such passageways are known *per se* from GB2,393,399 (Nasir), the text of which is hereby incorporated by reference and is intended to become an integral part of this disclosure. The gastric tube passageway, which is separate from the airway tube, runs from an opening 340 in the connector to an opening 341 in the distal tip of the device, and alongside the airway tube for the majority of its length. Alternatively, and not shown, the gastric tube passageway can run as a separate tube within the airway tube for a proportion of its length.

A further embodiment of the present invention is illustrated in Figures 25 to 32 inclusive. Figure 25 illustrates a perspective view of the dorsal side of the device. A similar numbering system to that used in Figures 1 to 8 has been used in these figures. The device 410 consists of an airway tube 411, the proximal end of which is adapted to fit over a connector of the type shown as 15 in Figure 1 or an equivalent. Formed around the distal end of the airway tube is a laryngeal cuff 414. A buccal cavity stabiliser 422 is formed around the airway tube in the region between the laryngeal cuff 414 and the proximal end of the device. Whilst a desirable feature, it will be appreciated that the buccal cavity stabiliser is optional, and not an essential feature, and such devices will function without any buccal cavity stabiliser.

In this example the front face of the laryngeal cuff 431A is formed from a first material of a first Shore hardness and the remainder of the device 431B shown in Figures 25 to 32 inclusive, with the exception of a dorsal protruberance or inflatable cuff described below, is formed from a second material of a second Shore hardness. It is preferred that the first and second materials are different, and have different Shore hardnesses. However, it will be understood that his is also not essential and the whole of the device, excluding the connector (not shown) could be made of a single material. Where two different materials are used then typical Shore hardnesses for the first and second material are as described above in relation to the embodiment shown in Figures 1 to 9 inclusive.

A further feature of the current embodiment is an inflatable dorsal cuff or protruberance 440, the construction of which is shown in more detail in Figure 26. This cuff consists of an inflation tube 432, a substantially or relatively rigid back plate and frame 451 and a resiliently deformable flexible back cushion, sealed to the back plate and frame around the perimeter of the back cushion in a substantially fluid-tight fashion, thus creating an inflatable cavity or cuff. Introduction of air or some other fluid through the inflation tube 432 causes the back cushion to expand away from the cuff body. A check valve in the inflation tube enables the cuff to hold a given inflation or a given deflation.

Once assembled, a portion of the inflation tube 432 is housed within the body of the device, with a proximal end of the airway tube exiting the body through an opening 454 as shown in Figure 26.

In terms of methods of manufacture, the inflatable dorsal cuff assembly can be formed as a unit and fitted into a correspondingly shaped cavity in the back of the device, threading the inflation tube 432 through an internal passageway provided and out of aperture 454. Alternatively, the remainder of the device can be moulded around a pre-formed dorsal cuff in a one shot or two shot moulding process as required.

The face of the laryngeal cuff in this embodiment shares the features of the embodiment shown in Figures 1 to 8 in the narrow V-shaped opening 420,421 at the proximal end of the cuff, the raised epiglottic support 416,418, and the elongate ellipse-shaped cuff face.

The incorporation of an inflatable dorsal cuff gives improved sealing in certain animal species such as large domestic animals such as dogs, larger animals such as horses and in some humans, particularly in paediatric use.

In summary, a new type of airway device has been designed for securing and maintaining a patent airway during routine and emergency anaesthesia for operations during spontaneous or Intermittent Positive Pressure Ventilation (IPPV). It incorporates a non-inflatable or inflatable cuff made of a soft material. It is expected to cause less compression trauma whilst in situ in animals than other devices. The design includes a buccal cavity stabiliser that will aid insertion and thus help reduce the potential for rotation and consequent malposition. In certain embodiments the laryngeal cuff on the device is non-inflatable and, in final production, will be formed from a soft, deformable material that can adapt and conform to the individual detail of the subject's laryngeal inlet to provide a satisfactory seal for ventilation.

The cross-section of the buccal stabiliser shape is widened, elliptical in profile, symmetrical and laterally flattened to mirror the base of the tongue providing good vertical stability upon insertion. The tube section is firmer than the soft bowl of the cuff of the device. The firmness of the tube section and its natural oropharyngeal curvature allows the device to be inserted by grasping the proximal end of the device to glide the leading edge against the hard palate into the pharynx. During initial dissection trials, it did not prove necessary to insert fingers or laryngoscopes into the mouth of the subject to achieve full insertion. The smooth moulded surface of the device from the tip of the bowl of the cuff and throughout the entire airway tube section helps the device to slide easily caudally along the hard palate, pharynx and hypopharynx. The face of the non-inflatable laryngeal cuff is shaped to form an anatomical fit over the laryngeal inlet of a small animal patient. The rostral aspect of the laryngeal bowl incorporates an epiglottic rest. This has been included to prevent the epiglottis of the subject obstructing the opening in the distal end of the airway tube in use, by retaining the epiglottis substantially clear of the opening in the face of the laryngeal cuff.

In relation to earlier designs:-
- The cuff section of the device has been considerably narrowed, to create a much slimmer device laterally.
- It was found that inclusion of a gastric channel (to allow suction of regurgitated material) as in the i-gel (RTM) design, was not always possible with the very narrow cuff versions, especially for the rabbit and cat designs.
- The lateral walls of the laryngeal section of the cuff were increased in thickness, to provide greater deflection of the epiglottis away from the open bowl of the cuff.
- The oesophageal section of the device was narrowed, tapered and elongated. This provided directional stability on insertion of the device and additional stability once in place.
- The oral section of the device was shortened considerably to account for the difference in body size and head shape from the paediatric situation.
- A lateral pharyngeal stabiliser 20,21 was formed from soft extensions around the airway tube wall. These achieved greater resistance to rotation and gave greater protection against the epiglottis down-folding into the airway.
- Small radial ridges were created in the proximal area of the tube, thus providing increased friction to prevent tube ties slipping during general anaesthesia. These changes create a very secure tie, and allow the anaesthetist to attach the device easily and securely.

These devices find application in both animal and human use, particularly in paediatrics. Without wishing to be constrained to any particular theory, it is believed that these devices form a seal by the soft tip of the device forming a seal in the top of the oesophagus, and the face of the cuff forming a seal in the region closely surrounding the laryngeal inlet of the patient.

## Claims

1. An airway device (10, 110, 410) for animal or human use, said device comprising an airway tube (11, 111, 411) having a distal end (13, 113) and a proximal end (12, 112), wherein the distal end of the airway tube is surrounded by a laryngeal cuff (14, 114, 414), and wherein the face (31A, 131A, 41, 431A) of the laryngeal cuff (14, 114, 414) is shaped to form an anatomical fit over the laryngeal inlet of an animal or human patient, and to form a seal with the laryngeal inlet of the patient, wherein the face (31 A, 131 A, 41, 431 A) of the cuff comprises an opening (17,117) and wherein the face (31A, 131A, 41, 431A) of the cuff is substantially elliptical in shape but the shape of the opening (17, 117) in the face of the cuff is non elliptical and the width of the opening at the proximal end of the face of the cuff is narrower than the width of the opening (17, 117) at the distal end (13, 113) of the airway tube (11, 111, 411) wherein the face of the laryngeal cuff incorporates an epiglottic support (16, 116), being so sized, shaped and positioned as to prevent the epiglottis of the patient obstructing the opening (17, 117) in the distal end (13, 113) of the airway tube (11, 111, 411) in use, by retaining the epiglottis substantially clear of the opening (17, 117) in the end of the airway tube (11, 111, 411) and wherein the airway tube (11, 111) has a height H and a width W and wherein the epiglottic support/rest (16, 116) comprises a region of the cuff or airway tube which stands above or proud of the general height H of the profile of the airway tube and **characterised in that** the face (31A, 131A, 41, 431A) of the laryngeal cuff (14, 114, 414) is a generally elongate substantially elliptical shape and the ratio A/B of the length of the major axis A of the ellipse to the length of the minor axis B of the ellipse is greater than 2.3 ± 10%.

2. An airway device (10, 110, 410) according to Claim 1 wherein the ratio A/B is greater than 2.5 ± 10% and preferably the ratio A/B is 2.6 ± 10%.

3. An airway device (10, 110, 410) according to Claim 1 wherein the narrow region of the opening (17, 117) in the face of the cuff provides the epiglottic support (16, 116).

4. An airway device (10, 110, 410) according to any preceding claim wherein the opening (17, 117) in the airway tube at the proximal end of the laryngeal cuff (14, 114, 414) is substantially V-shaped, the narrowing of the V-shaped opening acting as an epiglottic rest (16, 116) to prevent the epiglottis of the patient obstructing the distal end (13, 113) of the airway tube (11,111, 411) in use.

5. An airway device (10, 110, 410) according to any preceding claim wherein the dorsal or posterior face of the laryngeal cuff incorporates one or more protuberances (20, 21, 40, 440), said protuberances being sized and shaped such that they contact, in use, the back of the pharynx of the subject.

6. An airway device according to Claim 5 wherein the protuberances take the form of a soft raised cushion or an inflatable dorsal cuff (40, 440, 20, 21).

7. An airway device according to Claim 5 or Claim 6 wherein the protuberance (20, 21, 40, 440) is located opposite the proximal part of the opening (17, 117) in the cuff face.

## Patentansprüche

1. Eine Atemweg-Vorrichtung (10, 110, 410) zur Anwendung bei Menschen oder Tieren, wobei die Vorrichtung ein Atemweg-Rohr (11, 111, 411) mit einem distalen Ende (13, 113) und einem proximalen Ende (12, 112) aufweist, wobei das distale Ende des Atemweg-Rohres von einer Kehlkopf-Manschette (14, 114, 414) umgeben ist, und bei der die Stirnfläche (31A, 131A, 41, 431 A) der Kehlkopf-Manschette (14, 114, 414) so geformt ist, dass sie einen anatomischen Sitz über dem Kehlkopf-Einlass eines Tier- oder Menschen-Patienten bildet und eine Abdichtung mit dem Kehlkopf-Einlass des Patienten bildet, wobei die Stirnfläche (31A, 131A, 41, 431 A) der Manschette eine Öffnung (17, 117) aufweist, und bei der die Stirnfläche (31A, 131A, 41, 431 A) der Manschette eine im Wesentlichen elliptische Form aufweist, wobei jedoch die Form der Öffnung (17, 117) in der Stirnfläche der Manschette nicht-elliptisch ist und die Breite der Öffnung an dem proximalen Ende der Stirnfläche der Manschette schmaler als die Breite der Öffnung (17, 117) an dem distalen Ende (13, 113) des Atemweg-Rohres (11, 111, 411) ist, wobei die Stirnfläche der Kehlkopf-Manschette eine Kehldeckel-Halterung (16, 116) beinhaltet, die so bemessen, geformt und angeordnet ist, dass sie verhindert, dass der Kehldeckel des Patienten die Öffnung (17, 117) in dem distalen Ende (13, 113) des Atemweg-Rohres (11, 111, 411) im Gebrauch verdeckt, indem der Kehldeckel im Wesentlichen von der Öffnung (17, 117) in dem Ende des Atemweg-Rohres (11, 111, 411) fort gehalten wird, und wobei das Atemweg-Rohr (11, 111) eine Höhe H und eine Breite W aufweist, und wobei die Kehldeckel-Halterung/Abstützung (16, 116) einen Bereich der Manschette oder des Atemweg-Rohres umfasst, der über die allgemeine Höhe H des Profils des Atemweg-Rohres vorspringt oder vorsteht, und **dadurch gekennzeichnet, dass** die Stirnfläche (31A, 131A, 41, 431A) der Kehlkopf-Manschette (14, 114, 414) eine allgemein langgestreckte im Wesentlichen elliptische Form aufweist und dass das Verhältnis A/B der Länge der Hauptachse A der Ellipse zur Länge der kleineren Achse B der Ellipse größer als 2,3 ± 10% ist.

2. Eine Atemweg-Vorrichtung (10, 110, 410) nach Anspruch 1, bei der das Verhältnis A/B größer als 2,5 ± 10% ist und das Verhältnis A/B vorzugsweise 2,6 ± 10% ist.

3. Eine Atemweg-Vorrichtung (10, 110, 410) nach Anspruch 1, bei der der schmale Bereich der Öffnung (17, 117) in der Stirnfläche der Manschette die Kehldeckel-Halterung (16, 116) bildet.

4. Eine Atemweg-Vorrichtung (10, 110, 410) nach einem der vorhergehenden Ansprüche, bei der die Öffnung (17, 117) in dem Atemweg-Rohr an dem proximalen Ende der Kehlkopf-Manschette (14, 114, 414) im Wesentlichen V-förmig ist, wobei die Verschmälerung der V-förmigen Öffnung als eine Kehldeckel-Stütze (16, 116) wirkt, um im Gebrauch den Kehldeckel des Patienten an einem Verschließen des distalen Endes (13, 113) des Atemweg-Rohres (11, 111, 411) zu hindern.

5. Eine Atemweg-Vorrichtung (10, 110, 410) nach einem der vorhergehenden Ansprüche, bei der die dorsale oder posteriore Stirnfläche der Kehlkopf-Manschette ein oder mehrere Vorsprünge (20, 21, 40, 440) beinhaltet, wobei die Vorsprünge so bemessen und geformt sind, dass sie im Gebrauch mit der Rückseite des Rachens des Patienten in Kontakt kommen.

6. Atemweg-Vorrichtung nach Anspruch 5, bei der die Vorsprünge die Form eines weichen vorspringenden Kissens oder einer aufblasbaren dorsalen Manschette (40, 440, 20, 21) aufweisen.

7. Eine Atemweg-Vorrichtung nach Anspruch 5 oder 6, bei der der Vorsprung (20, 21, 40, 440) entgegengesetzt zu dem proximalen Teil der Öffnung (17, 117) in der Manschetten-Stirnfläche liegt.

## Revendications

1. Dispositif pour voies respiratoires (10, 110, 410) à usage animal ou humain, ledit dispositif comprenant un tube pour voies respiratoires (11, 111, 411) ayant une extrémité distale (13, 113) et une extrémité proximale (12, 112), dans lequel l'extrémité distale du tube pour voies respiratoires est entourée par un ballonnet pour larynx (14, 114, 414) et dans lequel la face (31A, 131A, 41, 431A) du ballonnet pour larynx (14, 114, 414) est formée pour former un ajustement anatomique sur l'entrée du larynx d'un patient animal ou humain, et pour former un joint d'étanchéité avec l'entrée du larynx du patient, dans lequel la face (31A, 131A, 41, 431A) du ballonnet comprend une ouverture (17, 117) et dans lequel la face (31A, 131A, 41, 431A) du ballonnet a une forme sensiblement elliptique mais la forme de l'ouverture (17, 117) dans la face du ballonnet n'est pas elliptique et la largeur de l'ouverture au niveau de l'extrémité proximale de la face du ballonnet est plus étroite que la largeur de l'ouverture (17, 117) au niveau de l'extrémité distale (13, 113) du tube pour voies respiratoires (11, 111,411), dans lequel la face du ballonnet pour larynx incorpore un support épiglottique (16, 116) qui est dimensionné, formé et positionné afin d'empêcher l'épiglotte du patient d'obstruer l'ouverture (17, 117) dans l'extrémité distale (13, 113) du tube pour voies respiratoires (11, 111, 411) à l'usage, en retenant l'épiglotte sensiblement dégagée de l'ouverture (17, 117) dans l'extrémité du tube pour voies respiratoires (11, 111, 411) et dans lequel le tube pour voies respiratoires (11, 111) a une hauteur H et une largeur W et dans lequel le support/appui épiglottique (16, 116) comprend une région du ballonnet ou tube pour voies respiratoires qui est au-dessus de ou fait saillie de la hauteur générale H du profil du tube pour voies respiratoires et **caractérisé en ce que** la face (31A, 131A, 41, 431A) du ballonnet pour larynx (14, 114, 414) a une forme sensiblement elliptique généralement allongée et le rapport A/B de la longueur de l'axe majeur A de l'ellipse sur la longueur de l'axe mineur B de l'ellipse est supérieur à 2,3 ± 10%.

2. Dispositif pour voies respiratoires (10, 110, 410) selon la revendication 1, dans lequel le rapport A/B est supérieur à 2,5 ± 10% et de préférence le rapport A/B est de 2,6 ± 10%.

3. Dispositif pour voies respiratoires (10, 110, 410) selon la revendication 1, dans lequel la région étroite de l'ouverture (17, 117) dans la face du ballonnet fournit le support épiglottique (16, 116).

4. Dispositif pour voies respiratoires (10, 110, 410) selon l'une quelconque des revendications précédentes, dans lequel l'ouverture (17, 117) dans le tube pour voies respiratoires au niveau de l'extrémité proximale du ballonnet pour larynx (14, 114, 414) est sensiblement en forme de V, le rétrécissement de l'ouverture en forme de V servant d'appui épiglottique (16, 116) pour empêcher l'épiglotte du patient d'obstruer l'extrémité distale (13, 113) du tube pour voies respiratoires (11, 111, 411), à l'usage.

5. Dispositif pour voies respiratoires (10, 110, 410) selon l'une quelconque des revendications précédentes, dans lequel la face dorsale ou postérieure du ballonnet pour larynx incorpore une ou plusieurs protubérances (20, 21, 40, 440), lesdites protubérances étant dimensionnées et formées de sorte qu'elles sont en contact, à l'usage, avec l'arrière du pharynx du patient.

6. Dispositif pour voies respiratoires selon la revendication 5, dans lequel les protubérances prennent la forme d'un coussin relevé souple ou d'un ballonnet dorsal gonflable (40, 440, 20, 21).

7. Dispositif pour voies respiratoires selon la revendication 5 ou la revendication 6, dans lequel la protubérance (20, 21, 40, 440) est positionnée à l'opposé de la partie proximale de l'ouverture (17, 117) dans la face du ballonnet.
